# EUROPEAN PATENT APPLICATION

(11) **EP 4 488 263 A2**
(43) Date of publication of application: **08.01.2025**
(21) Application number: 23763046.2
(22) Date of filing: 02.03.2023
(51) Int. Cl.: C07D 211/46, C07D 413/12, A61K 31/5377, A61P 25/00

(54) **PHENYLUREA DERIVATIVE AND PHARMACEUTICAL USE THEREOF**

(30) Priority: 02.03.2022 CN 202210200080
(71) Applicant: Hangzhou Bio-Sincerity Pharma-tech Co., Ltd., Hangzhou, Zhejiang 311100 (CN)
(72) Inventor: FENG, Enguang, Hangzhou, Zhejiang 311100 (CN); QIN, Jinjing, Hangzhou, Zhejiang 311100 (CN); JIN, Lifeng, Hangzhou, Zhejiang 311100 (CN); JIN, Zewu, Hangzhou, Zhejiang 311100 (CN); LI, Yuanyuan, Hangzhou, Zhejiang 311100 (CN); XIONG, Xiaohong, Hangzhou, Zhejiang 311100 (CN); LEI, Shaowei, Hangzhou, Zhejiang 311100 (CN); SHEN, Ximing, Hangzhou, Zhejiang 311100 (CN); LOU, Jinfang, Hangzhou, Zhejiang 311100 (CN)
(74) Representative: Valet Patent Services Limited
(86) International application number: PCT/IB2023/000143
(87) International publication number: WO 2023/166349

(57) **Abstract**

The present invention discloses a phenylurea derivative, and specifically relates to the compound of free base, or isomer, or solvate, or pharmaceutically acceptable salt thereof; methods of preparing compound; compound composition and therapeutic uses thereof. The present invention has developed a series of structurally novel compounds based on histamine H3 receptor ligands, and a series of relevant biological tests have been performed on the compounds. The results of the tests all indicate that the compounds have significant H3 receptor antagonistic activity and can be used as a lead compound for the prevention or treatment of H3 receptor-related diseases.

## Description

### FIELD OF THE INVENTION

The present invention belongs to the field of pharmaceutical technology and relates to a phenylurea derivative and pharmaceutical use thereof, and specifically relates to the compound of free base, or isomer, or solvate, or pharmaceutically acceptable salt thereof, preparation methods of the compound, compositions and therapeutic uses thereof.

### BACKGROUND OF THE INVENTION

Following the Human Genome Project, the histamine receptor family has expanded to four distinct G protein-coupled receptors (GPCRs): the H1, H2, H3, and H4 receptors (Nature Review Drug Discovery, 2005, 4, 107-120). Histamine H3 receptors are mainly expressed in the central nervous system and less frequently in the peripheral nervous system.

Histamine H3 receptor (H3R) is widely expressed in the brain, mainly in the cerebral cortex, hippocampus, amygdala, striatum and other areas closely related to memory and cognitive abilities. Blocking these receptors with selective antagonists/inverse agonists can increase the release of neurotransmitters such as acetylcholine, dopamine or 5-hydroxytryptamine, thereby regulating a variety of neuropathic behaviors such as learning and memory, wakefulness and sleep (British Journal of Pharmacology, 2008, 154(6), 1166-1181).

Literatures suggests that ligands of histamine H3 receptor can be used to treat cognitive impairment (British Journal of Pharmacology, 2008, 154(6), 1166-1181), dementia (Drug News Perspective, 2010, 23(2), 99-103), attention deficit hyperactivity disorder, obesity (Indian Journal of Pharmacology, 2001, (33), 17-28), schizophrenia (Biochemical Pharmacology, 2007, 73(8), 1215-1224), and pain (Journal of Pharmacology and Experimental Therapeutics, 2011, 336(1), 30-37).

Presently, histamine H3 receptor ligands compounds are mainly divided into two categories: imidazole ring substituted at the 4 (5) position and non-imidazole ring. Compounds containing imidazole rings have the disadvantages of low blood-brain barrier penetration, interaction with cytochrome P-450 proteins, liver toxicity, and ocular toxicity. Representative patents of non-imidazole ring compounds: ①US20020177589A1 discloses new ligands that can be used to regulate histamine H3 receptor. The compounds in this patent can be used to treat Alzheimer's disease and cognitive impairment. The representative compound is ABT-239, which has serious cardiac side effects in clinical trials, resulting in the termination of clinical trials. ②WO2012114348A1 discloses novel compounds as ligands of histamine H3 receptor, which can be used to treat various diseases, such as cognitive impairment, dementia, etc. Samelisant (SUVN-G3031), an H3R antagonist/inverse agonist for the treatment of cognitive impairment, is currently under development. The drug's Phase II clinical indication is narcolepsy, and the Phase I clinical trial for cognitive impairment was conducted from 2015 to 2017, with no subsequent progress. ③WO2014030170A1 discloses pyridazinone derivatives as histamine H3 inhibitor, as well as their uses, preparation methods and pharmaceutical compositions. The inventive compounds can be used to treat various diseases such as cognitive impairment, sleep/wakefulness disorders, eating disorders, etc., but the clinical trial was terminated due to the unsatisfactory PK/PD data.

In summary, while a number of histamine H3 receptor ligands analogs are disclosed, only Pitolisant (H3R antagonist/inverse agonist) has been approved for marketing in this area of research and development to date, with the marketed indication of narcolepsy. SUVN-G3031 and other investigational drugs in Phase 2 clinical studies are still narcolepsy, the clinical studies in neurological disorders such as cognitive impairment have been declared failures.

Therefore, there is an urgent need to discover a new compound with novel structure, high safety profile, and better drug's efficacy than Pitolisant and SUVN-G3031, which is used to prevent or treat a disease associated with H3 targets. This is still not solved by those skilled in the art.

### SUMMARY OF THE INVENTION

The present invention aims to provide at least one structurally novel phenylurea derivative that can be used as a histamine H3 receptor ligands compound to prevent or treat histamine H3 receptor-related diseases.

To achieve the above-mentioned invention purpose, the technical solution provided by the present invention is as follows:
A phenylurea derivative, which is a compound of general formula I or a pharmaceutically acceptable salt thereof: 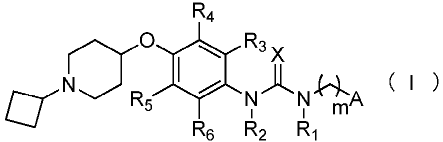 wherein:
X is selected from O or S;
m is selected from 0, 1, 2, 3, 4, 5 or 6;
A is selected from C₁₋₆ alkyl, -NR'R", substituted or unsubstituted 5-8 membered heterocyclyl, and substituted or unsubstituted 5-8 membered heteroaryl; wherein the heterocyclyl or heteroaryl has at least one substituent selected from one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₁ and R₂ are each independently selected from hydrogen, C₁₋₆ alkyl, -C(O)-alkyl or -S(O)₂-alkyl;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, R₅ and R₆ together with the carbon atoms on the benzene ring to which they are attached form a substituted or unsubstituted benzobicyclic structure, wherein the benzobicyclic structure can be but not limited to, a benzoheterocyclic ring;
the heterocyclyl, heteroaryl and benzoheterocyclic group contain at least one heteroatom, and the heteroatom is selected from N, O or S.

Preferably, a compound of general formula II or a pharmaceutically acceptable salt thereof: 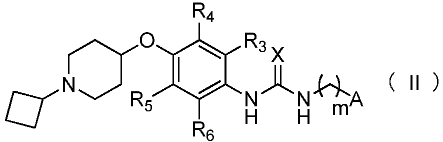 wherein:
X is selected from O or S;
m is selected from 0, 1, 2, 3, 4, 5 or 6;
A is selected from substituted or unsubstituted 5-8 membered heterocyclyl, and substituted or unsubstituted 5-8 membered heteroaryl; wherein the heterocyclyl or heteroaryl has at least one substituent selected from one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, R₅ and R₆ together with the carbon atoms on the benzene ring to which they are attached form a substituted or unsubstituted benzobicyclic structure, wherein the benzobicyclic structure can be but not limited to, a benzoheterocyclic ring;
the heterocyclyl, heteroaryl and benzoheterocyclic group contain at least one heteroatom, and the heteroatom is selected from N, O or S.

Preferably, a compound of general formula II-1 or a pharmaceutically acceptable salt thereof: 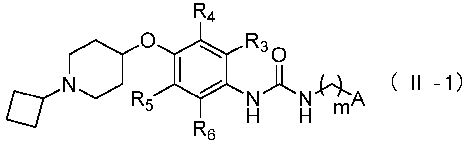 wherein:
m is selected from 0, 1, 2, 3, 4, 5 or 6;
A is selected from one of substituted or unsubstituted 6-membered heterocyclyl and substituted or unsubstituted 6-membered heteroaryl; wherein the heterocyclyl or heteroaryl has at least one substituent selected from one or more of C₁₋₆ alkyl, C₁₋₆ alkoxy, halogen, amino, hydroxyl, C₁₋₆ haloalkyl, C₁₋₆ alkylhydroxyl, C₁₋₆ alkylamino, C₁₋₆ haloalkoxy, C₁₋₆ alkoxyhydroxyl and C₁₋₆ alkoxy amino;
R₃, R₄, R₅, and R₆ are each independently selected from one or more of hydrogen, halogen, C₁₋₆ alkyl, amino, hydroxyl, C₁₋₆ haloalkyl, C₁₋₆ alkylhydroxyl and C₁₋₆ alkylamino,;
the heterocyclyl and heteroaryl contain at least one heteroatom, and the heteroatom is selected from N, O or S.

Preferably, a compound of general formula III or a pharmaceutically acceptable salt thereof: 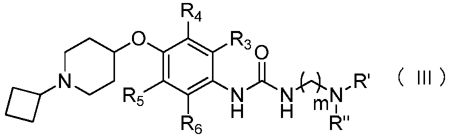 wherein:
m is selected from 0, 1, 2, 3, 4, 5 or 6;
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, R₅ and R₆ together with the carbon atoms on the benzene ring to which they are attached form a substituted or unsubstituted benzobicyclic structure, wherein the benzobicyclic structure can be but not limited to, a benzoheterocyclic ring;
the benzoheterocyclic group contains at least one heteroatom, and the heteroatom is selected from N, O or S.

Preferably, a compound of general formula IV or a pharmaceutically acceptable salt thereof: 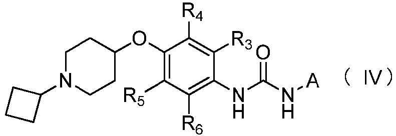 wherein:
A is selected from C₁₋₆ alkyl;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, R₅ and R₆ together with the carbon atoms on the benzene ring to which they are attached form a substituted or unsubstituted benzobicyclic structure, wherein the benzobicyclic structure can be but not limited to, a benzoheterocyclic ring;
the benzoheterocyclic group contains at least one heteroatom, and the heteroatom is selected from N, O or S.

The present invention also provides phenylurea derivatives, including a series of compounds as numbered in BIOS-A-1 to BIOS-A-23, or isomers thereof, or solvates thereof, or pharmaceutically acceptable salts thereof:
BIOS-A-1: 1-(4-((1-cyclobutylpiperidin-4-yl) oxy) phenyl)-3-cyclopentylurea;
BIOS-A-2:1-(4-((1-cyclobutylpiperidin-4-yl)oxy)phenyl)-3-cyclohexylurea;
BIOS-A-3 : 1-(4-((1-cyclobutylpiperidin-4-yl)oxy)phenyl)-3-(2-morpholinoethyl)urea;
BIOS-A-4: 1-(4-((1-cyclobutylpiperidin-4-yl)oxy)phenyl)-3-(2-(piperidin-1-yl)ethyl)urea;
BIOS-A-5: 1-(4-((1-cyclobutylpiperidin-4-yl)oxy)phenyl)-3-(2-(pyrrolidin-1-yl)ethyl)urea;
BIOS-A-6: (*R*)-1-(4-((1-cyclobutylpiperidin-4-yl)oay)phenyl)-3-(2-(2-methylpyrrolidin-1-yl)ethyl)urea;
BIOS-A-7: 1-(4-((1-cyclobutylpiperidin-4-yl)oxy)phenyl)-3-(2-(4,4-difluoropiperidin-1-yl)ethyl)urea;
BIOS-A-8: 1-(4-((1-cyclobutylpiperidin-4-yl)oxy)phenyl)-3-(2-thiomorpholinoethyl)urea;
BIOS-A-9:1-(4-((1-cyclobutylpiperidin-4-yl)oxy)phenyl)-3-(2-(4-hydroxypiperidin-1-yl)ethyl)urea;
BIOS-A-10: 1-(4-((1-cyclobutylpiperidin-4-yl)oay)phenyl)-3-(2-(4-methylpiperazin-1-yl)ethyl)urea;
BIOS-A-11: 1-(4-((1-cyclobutylpiperidin-4-yl)oxy)phenyl)-3-(2-(4-isopropylpiperazin-1-yl)ethyl)urea;
BIOS-A-12: 1-(4-((1-cyclobutylpiperidin-4-yl)oxy)phenyl)-3-(2-(3-hydroxypiperidin-1-yl)ethyl)urea;
BIOS-A-13: 1-(4-((1-cyclobutylpiperidin-4-yl)oxy)phenyl)-3-(2-(N-Methylhomopiperazin-1-yl)ethyl)urea;
BIOS-A-14: 1-(4-((1-cyclobutylpiperidin-4-yl)oay)phenyl)-3-(2-(piperazin-1-yl)ethyl)urea;
BIOS-A-15:1-(2-(4-cyclobutylpiperazin-1-yl)ethyl)-3-(4-((1-cyclobutylpiperidin-4-yl)oxy)phenyl)urea;
BIOS-A-16: 1-(4-((1-cyclobutylpiperidin-4-yl)oxy)phenyl)-3-isopropylurea;
BIOS-A-17: 1-(4-((1-cyclobutylpiperidin-4-yl)oxy)phenyl)-3-(2-(dimethylamino)ethyl)urea;
BIOS-A-18: 1-(4-((1-cyclobutylpiperidin-4-yl)oxy)phenyl)-3-(2-(diethylamino)ethyl)urea;
BIOS-A-19: 1-(4-((1-cyclobutylpiperidin-4-yl)oxy)phenyl)-3-(2-(ethylamino)ethyl)urea;
BIOS-A-20: 1-(4-((1-cyclobutylpiperidin-4-yl)oxy)phenyl)-3-(2-morpholinoethyl)thiourea;
BIOS-A-21: 1-(4-((1-cyclobutylpiperidin-4-yl)oay)phenyl)-3-(2-(piperidin-1-yl)ethyl)thiourea;
BIOS-A-22: 1-(4-((1-cyclobutylpiperidin-4-yl)oxy)phenyl)-3-(2-(pyrrolidin-1-yl)ethyl)thiourea; and
BIOS-A-23: (*R*)-1-(4-((1-cyclobutylpiperidin-4-yl)oxy)phenyl)-3-(2-(2-methylpyrrolidin-1-yl)ethyl)thiourea.

The compounds as numbered BIOS-A-1 to BIOS-A-23, or pharmaceutically acceptable salts thereof, may have the following structural formula:

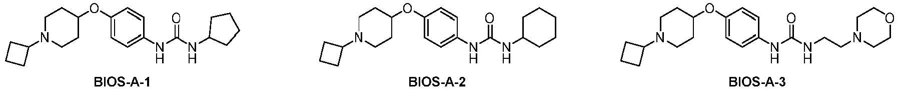

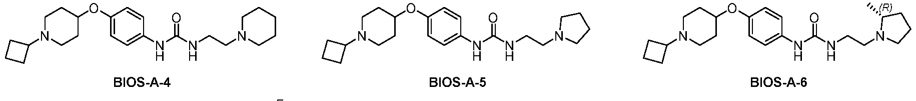

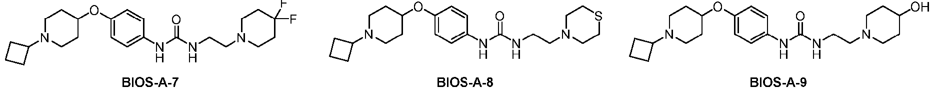

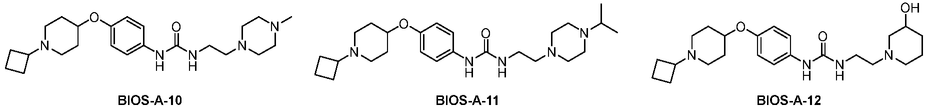

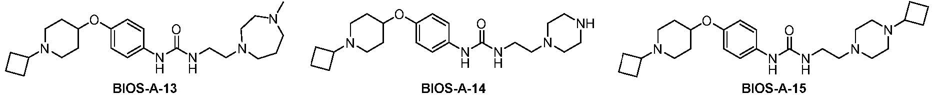

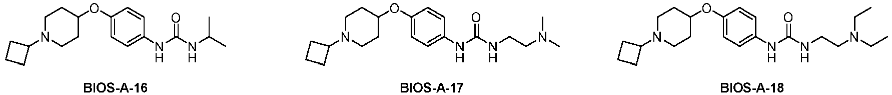

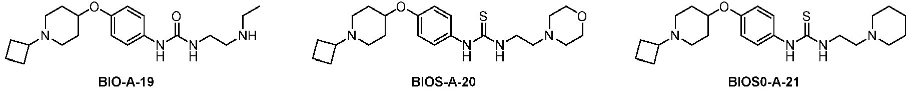

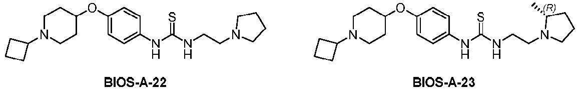

Furthermore, the present invention also provides phenylurea derivatives, which are chiral compounds as follows or a pharmaceutically acceptable salt thereof:
(*R*)-1-(4-((1-cyclobutylpiperidin-4-yl)oxy)phenyl)-3-(2-(2-methylpyrrolidin-1-yl)ethyl)urea; and
(*R*)-1-(4-((1-cyclobutylpiperidin-4-yl)oxy)phenyl)-3-(2-(2-methylpyrrolidin-1-yl)ethyl)thiourea.

The "compound" described in the present invention includes but is not limited to the following forms of the compounds: free base, stereoisomers, geometric isomers, tautomers, isotopes, pharmaceutically acceptable salts, solvates, hydrates, prodrugs (esters or phosphates), etc.

The "compound" of the present invention can be asymmetric, for example, having one or more stereoisomers. Unless otherwise indicated, all stereoisomers are included enantiomers and diastereomers. The compounds containing asymmetric carbon atoms of the present invention can be isolated in optically pure form or in racemic form. Optically pure forms can be obtained by resolution of racemic mixtures, synthesis using chiral starting materials or chiral reagents.

The "pharmaceutically acceptable salt" of the present invention refers to salt of the compounds that prepared from the compounds with specific substituents and relatively non-toxic bases. When compounds contain relatively acidic functional groups, the bases addition salt of these compounds can be obtained by contacting the compound with a sufficient amount of base in pure solution or suitable inert solvents. Pharmaceutically acceptable base addition salts, including but not limited to sodium, potassium, calcium, magnesium salts, ammonium, or organic ammonia. Such as: alkali metal salts, alkaline earth metal salts, other metal salts, inorganic alkali salts, organic alkali salts, inorganic acid salts, lower alkane sulfonates, aryl sulfonates, organic acid salts, and amino acid salts.

In addition to the salt forms, the compounds provided herein exist in prodrug forms. The prodrugs of the compounds described herein are readily converted to the compounds of the present invention by chemical changes under physiological conditions. In addition, precursor drugs can be converted to the compounds of the invention in vivo by chemical or biochemical methods.

The terms used in this article have the following meanings for compounds containing the above general structure:
The term "halogen" refers to fluorine, chlorine, bromine or iodine, preferably fluorine, chlorine or bromine.

The term "alkyl" refers to straight-chain or branched saturated hydrocarbon groups consisting of carbon atoms and hydrogen atoms, such as C₁₋₆ alkyl, including but not limited to methyl, ethyl, propyl (including n-propyl and isopropyl), butyl (including n-butyl, isobutyl, sec-butyl or tert-butyl), pentyl (including n-pentyl, isopentyl, neopentyl), n-hexyl, and 2-methylhexyl.

The term "cycloalkyl" refers to monocyclic or bicyclic alkyl groups composed of carbon atoms and hydrogen atoms, such as C₃₋₈ cycloalkyl groups, including but not limited to cyclopropyl, cyclobutyl, cyclopentyl, cyclohexyl, cycloheptyl and cyclooctyl.

The term "alkoxy" refers to straight or branched alkyl groups connected by oxygen atoms, such as C₁₋₆ alkoxy, including but not limited to methoxy, ethoxy, n-propoxy (including *n*-propoxy and isopropoxy), butoxy (including *n*-butoxy, isobutoxy, sec-butoxy or tert-butoxy), pentoxy (including *n*-pentoxy, isopentoxy, neopentoxy), n-hexyloxy, 2-methylhexyloxy, etc.

The term "alkylamino" refers to open-chain alkyl groups containing nitrogen atoms, such as C₁₋₆ alkylamino, including but not limited to methylamino, ethylamino, isopropylamino, dimethylamino, methylethylamino, diethylamino, etc.

The term "aryl" refers to all-carbon monocyclic or condensed polycyclic groups of 5-12 (integer) carbon atoms, having a completely conjugated π-electron system, including but not limited to benzene rings, naphthalene rings, and anthracene rings.

The terms "heterocyclyl" and "heteroaryl" refer to monocyclic or condensed rings having 3-12 (integer) ring atoms, wherein 1, 2, 3 or more ring atoms are selected from one or more of N, O and S, and the remaining ring atoms are C, and have a completely conjugated or unconjugated π-electron system. The heterocyclic group can be saturated or unsaturated groups. Examples of heterocyclic groups include, but are not limited to, pyrrolyl, indolyl, pyrrolidinyl, imidazolyl, pyrazolyl, tetrazolyl, pyridyl, quinolyl, isoquinolyl, piperidyl, pyrimidyl, pyrazinyl, piperazinyl, furanyl, pyranyl, morpholinyl.

The present provides a method of preparing the above compounds by the following steps, but not limited to the following methods:

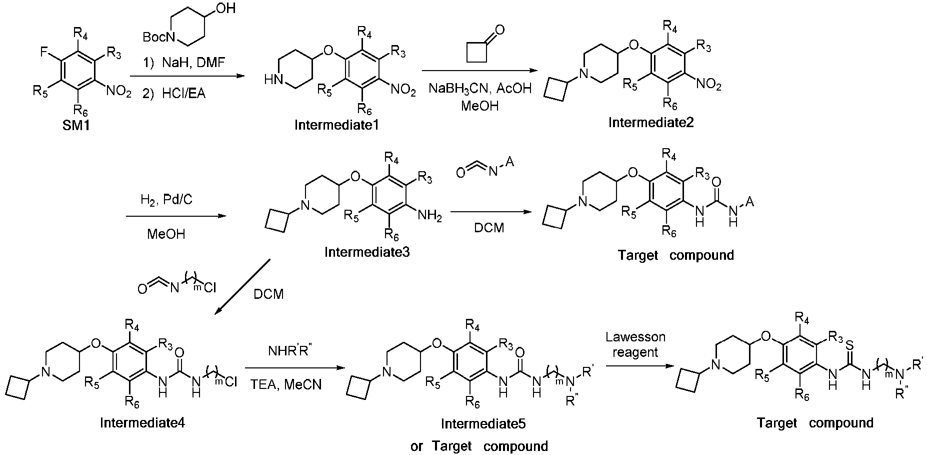

The corresponding reaction procedure is shown as follows:
The para-fluorinated nitrobenzene compound (starting material 1), piperidinol and sodium hydride were dissolved in DMF and then reacted in hydrochloric acid/ethyl acetate solution to obtain intermediate 1. Then intermediate 1 underwent reductive amination reaction with o-cyclobutanone to obtain intermediate 2. Intermediate 2 was reduced in the Pd/C hydrogen atmosphere to obtain intermediate 3. And intermediate 3 was reacted with isocyanate in dichloromethane to obtain the target compound;

Alternatively, intermediate 3 was reacted with chloroisocyanate in dichloromethane to obtain intermediate 4, which was then reacted with amine to obtain intermediate 5 or the target compound;

Alternatively, intermediate 5 was reacted with Lawesson's reagent to obtain the target compound.

The substituents R', R", R₃, R₄, R₅, and R₆ involved in the above-mentioned synthesis method are defined as follows:
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, oRs and R₆ together with the carbon atoms on the benzene ring to which they are attached form a substituted or unsubstituted benzobicyclic structure, wherein the benzobicyclic structure can be but not limited to, a benzoheterocyclic ring;

The invention also provides pharmaceutical compositions comprising at least one compound as described above or pharmaceutically acceptable salts thereof as active ingredients, and at least one or more pharmaceutically acceptable carriers.

The "pharmaceutical composition" of the invention refers to a formulation of one or more compounds of the present invention or salts thereof and carrier generally accepted in the art for delivery of biologically active compounds to an organism (e.g., human). The purpose of pharmaceutical compositions is to facilitate administration and delivery to theorganism.

The routes of administration of the compounds or pharmaceutically acceptable salts thereof, or pharmaceutical compositions thereof described herein, including but are not limited to, oral, rectal, transmucosal, trans-intestinal, topical, transdermal, inhalation, parenteral, sublingual, intravaginal, intranasal, intraocular, intraperitoneal, intramuscular, subcutaneous, and intraventricular administration. The preferred route of administration is oral administration.

The present invention also provides the uses of preparing compounds or pharmaceutical compositions as described hereinbefore in the prevention or treatment of diseases associated with histamine H3 receptors.

Preferably, the foregoing medications are used for the prevention or treatment of cognitive disorders, dementia, attention deficit hyperactivity disorder, schizophrenia, epilepsy, sleep disorders, sleep apnea, obesity, eating disorders, pain or pruritus.

Preferably, the foregoing medications are used for the prevention or treatment of neuropathic pain, which can be but not limited to, peripheral neuropathic pain or central neuropathic pain.

Preferably, the peripheral neuropathic pain is trigeminal neuralgia, glossopharyngeal neuralgia, acute or chronic inflammatory demyelinating polyradiculoneuritis, alcoholic polyneuropathy, chemotherapy-induced polyneuropathy, complex regional pain syndrome, entrapment neuralgia (such as carpal tunnel syndrome), HIV sensory neuralgia, iatrogenic neuralgia (such as pain after mastectomy), tumor compression or infiltration neuralgia, nutritional deficiency-related neuralgia, diabetic neuralgia, phantom limb pain, post-herpetic neuralgia, post-radiotherapy plexus pain, radiculopathy (cervical, thoracic or lumbar sacral), toxic exposure-related neuralgia or post-traumatic neuralgia.

Preferably, the central neuropathic pain is post-stroke pain, multiple sclerosis-related pain, Parkinson's disease-related pain, post-traumatic spinal cord injurious pain, syringomyelia, post-ischemic myelopathy, compressive myelopathy, HIV myelopathy or post-radiation myelopathy.

Compared with the prior arts, the present invention has the following beneficial effects:
The present invention has developed a series of phenylurea derivatives with novel structures based on histamine H3 receptor ligands. The series of compounds are characterized by simple preparation routes and easy availability of raw materials.

The results of the series of related biological tests all indicate that the compounds have significant H3 receptor antagonistic activity and can be used as lead compounds to prevent or treat a disease associated with histamine H3 receptor

### DESCRIPTION OF THE DRAWINGS

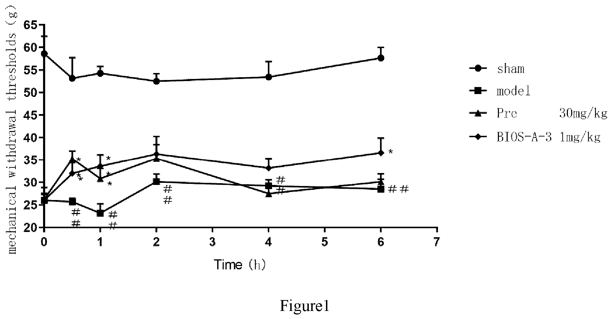

Figure 1 shows the graphs of the changes in mechanical withdrawal threshold of each experimental group at different times and different doses.

In the figure, compared with the MODEL group, *, P<0.05; **, P<0.01; compared with the SHAM group, ^{#}, P<0.05; ^{##}, P<0.01.

### EMBODIMENTS OF THE INVENTION

The following are specific embodiments of the present invention to further describe the technical solution, but the protection scope of the present invention is not limited to these embodiments. Any changes or equivalent substitutions that do not deviate from the concept of the present invention are included in the protection scope of the present invention.

The solvents, equipment, codes and full names involved in the embodiments of the present invention are as follows:
Code Bn: refers to benzyl;
Code DMF: refers to *N,N-*dimethylformamide;
Code TFA: refers to trifluoroacetic acid;
Code TsCl: refers to *p*-toluenesulfonyl chloride;
Code TEA: refers to triethylamine;
Code DCM: refers to dichloromethane;
Code Boc: refers to tert-butyloxycarbonyl;
Code Lawesson reagent: refers to 2,4-bis(p-methoxyphenyl)-1,3-dithiodiphosphatane-2,4-sulfide.

In the methods for preparing the target compounds provided by the present invention: the liquid chromatography uses the Waters Symmetry C18 chromatographic columns. TLC is performed using GF254 (0.25 mm). Nuclear magnetic resonance spectroscopy (NMR) is measured by the Bruker-400 NMR spectrometer. Liquid chromatography-mass spectrometry (LC/MS) is performed by the Waters ZQ mass spectrometer detector (column: Waters Symmetry C18, mm, 5 µm, 35 °C) in ESI (+) ion mode.

In addition, all operations involving easily oxidized or hydrolyzed starting materials are carried out under nitrogen protection. Unless otherwise specified, the starting materials used in the present invention are commercially available and can be used directly without further purification.

The starting materials, common intermediates, etc. involved in the embodiments of the present invention can be purchased commercially or obtained by self-production. Among them, the starting materials and common intermediates that need to be obtained by self-production are prepared in detail as follows.

### I. Preparation of common intermediate A1:

The synthesis route of intermediate A1 is as follows:

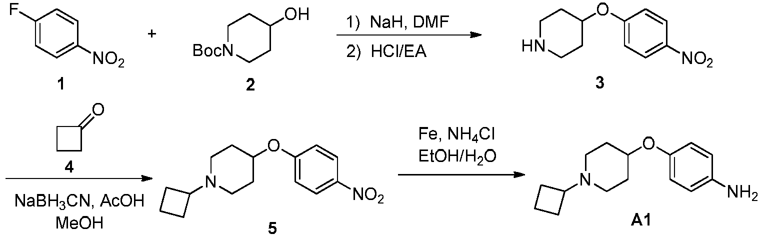

### Step 1: Synthesis of 4-(4-nitrophenoxy) piperidine (3)

*N*-Boe-4-hydroxypiperidine (compound 2, 5.14 g, 25.51 mmol) was dissolved in anhydrous *N*,*N-*dimethylformamide (40 mL) under nitrogen atmosphere in a 500 mL bottle. Sodium hydride (1.02 g, 25.51 mmol) was added and incubated at 0 °C for 1 hour. A solution of 4-fluoronitrobenzene (compound 1, 3.0 g) in anhydrous *N,N*-dimethylformamide (5 mL) was added dropwise. The mixture was alow to react at room temperature for 6 hours. When the reaction completed, the reaction was quenched by water (320 mL), extracted three times with ethyl acetate, and the organic phases were combined, washed by saline, dried over anhydrous sodium sulfate, filtered. The filtrate was concentrated to afford 9.56 g crude brown liquid.

The crude was dissolved in HCl/EA (30 mL) and reacted at room temperature for 1-2 hours. When the reaction completed, filter the reaction, affording 5.55g white solid (compound 3), with a yield of 84.10%.

MS:223.1[M+H]+_{∘}

### Step 2: Synthesis of 1-cyclobutyl-4-(4-nitrophenoxy)piperidine (5)

Compound 3 (5.55 g, 21.45 mmol) and 3 mL triethylamine were dissolved in 40 mL anhydrous methanol, and then cyclobutanone (compound 4, 2mL) and acetic acid (2 mL) were added. The reaction was carried out under ice bath for 1 hour before 2.1 g sodium cyanoborohydride was added. Then the mixture was reacted overnight at room temperature for 12 hours. The reaction of the raw material was complete as detected by thin layer chromatography.

The reaction solution was concentrated under reduced pressure and alkalized with sodium carbonate solution to pH 8-9. The mixture was extracted three times with ethyl acetate, and combined the organic phases. The organic phase was washed by saline, dried over anhydrous sodium sulfate, and concentreted to afford 6.77 g yellow solid (compound 5), with a yield of 99.0%.

MS:277.1[M+H]+_{∘}

### Step 3: Synthesis of 4-((1-cyclobutylpiperidin-4-yl)oxy)aniline (A1)

Compound 5 (5.77 g, 24.50 mmol) was dissolved in 180 mL ethanol, followed by the addition of 9.6 g reduced iron powder, 2.36 g ammonium chloride, and 60 mL water. The reaction was carried out in 80 °C oil bath for 2-3 hours. The reaction of the raw material was complete as detected by thin layer chromatography.

The reaction solution was filtered through diatomaceous earth and the residue was washed with ethanol 4-5 times. The mixture was extracted three times with ethyl acetate, and the organic phases were combined. The organic phase was washed by saline, dried with anhydrous sodium sulfate, and concentrated affording 4.99 g yellow brown liquid (Intermediate A1), with a yield of 82.68%.

MS:247.1[M+H]+_{∘}

### II. Preparation of common intermediate A2:

The synthesis route of intermediate A2 is as follows:

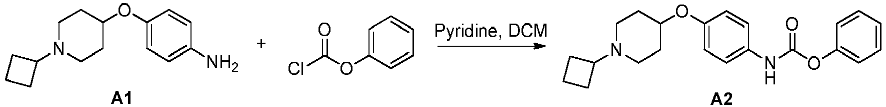

Intermediate A1 (300 mg, 1.242 mmol) and pyridine (150 mL, 1.491 mmol) were dissolved in 8 mL anhydrous dichloromethane, reacted under ice bath for 10 min. The mixture reacted at room tempreture for 2-3 hours after the addition of 187 µL benzyl chloroformate. The reaction of the raw material was complete as detected by thin layer chromatography.

The reaction solution was concentrated to dryness and alkalized with sodium carbonate solution to pH 8-9. The mixture was extracted three times with ethyl acetate, and combined the organic phases. The organic phase was washed by saline, dried over anhydrous sodium sulfate, and concentreted to afford 480 mg brown crude product (Intermediate A2), with a yield of 100%.

MS:367.2[M+H]⁺_{∘}

### III. Preparation of common intermediate A3:

The synthesis route of intermediate A3 is as follows:

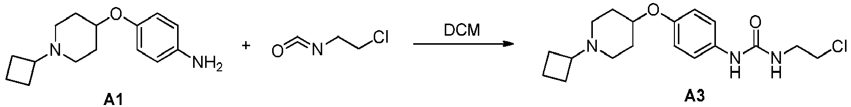

Intermediate A1 (2.01 g, 8.16 mmol) was dissolved in 20 mL anhydrous dichloromethane, and the mixture was reacted under icebath for 10 min. The reaction was carried out at room temperature for 3-4 hours after 835 µL chloroethyl isocyanate was added dropwise. The reaction of the raw material was complete as detected by thin layer chromatography.

The reaction solution was filtered, and the residue was washed with a small amount of dichloromethane, and the residue was dried, affording 1.96 g white powder (Intermediate A3), with a yield of 68.26%.

MS:352.3[M+H]⁺_{∘}

The following Example 1 to 23 are the preparation examples of the free base and/or hydrochloride structure of the BIOS-A series compounds described in the present invention. It should be noted that inaddition to the hydrochloride from mentioned in the Examples, BIOS-A series compounds can also be prepared in other pharmaceutically acceptable salt forms, including but not limited to sulfates, nitrates, nitrites, maleic acid salts, fumarates, formatted, diformes, and acetates, etc. The preparation of these compounds can be inspired by the corresponging technical insights provided in Examples 1 to 23 of the present invention.

### Example 1: Preparation of 1-(4-((1-cyclobutylpiperidin-4-yl) oxy) phenyl)-3-cyclopentylurea (BIOS-A-1)

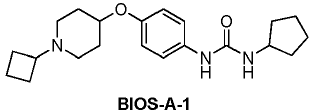

The synthesis route is as follows:

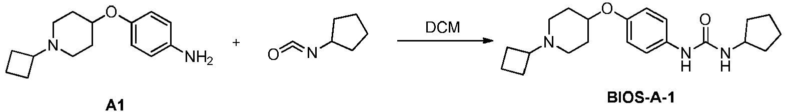

Intermediate A1 (100mg, 0.406 mmol) and cyclopentyl isocyanate (46 µL,0.406 mmol) were dissolved in 4mL dichloromethane and reacted at room temperature for 4-5 hours. The reaction of the raw material was complete as detected by thin layer chromatography.

The reaction solution was filtered, and the residue was washed with a small amount of dichloromethane, affording 90 mg white solid (compound BIOS-A-1), with a yield of 62.00%
MS:358.1[M+H]⁺_{∘}
¹H NMR (400 MHz, DMSO-*d*₆) δ 8.07 (s, 1H), 7.24 (d, *J* = 8.6 Hz, 2H), 6.80 (d, *J* = 8.6 Hz, 2H), 6.06 (d, *J* = 7.2 Hz, 1H), 4.27∼4.11 (m, 1H), 3.98∼3.85 (m, 1H), 2.73∼2.62 (m, 1H), 2.05∼1.46 (m, 20H), 1.39∼1.28 (m, 2H).

### Example 2: Preparation of 1-(4-((1-cyclobutylpiperidin-4-yl)oxy)phenyl)-3-cyclohexylurea (BIOS-A-2)

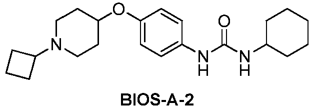

The synthesis route is as follows:

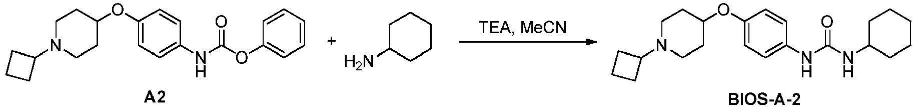

Intemediate A2 (260 mg, 0.709 mmol), cyclohexylamine (97 µL, 0.851 mmol), and triethylamine (197 µL) were dissolved in 4 mL acetonitrile and reacted overnight at room temperature. The reaction of the raw material was complete as detected by thin layer chromatography.

The reaction solution was concentrated to dryness, and the crude product was purified using a preparative plate (petroleum ether: ethyl acetate: triethylamine = 1: 1: 0.1), affording 116 mg yellow solid (compound BIOS-A-2), with a yield of 44.04%.
MS:372.5[M+H]⁺_{∘}
¹H NMR (400 MHz, DMSO-*d*₆) δ 8.61 (s, 1H), 7.29 (d, *J* = 8.6 Hz, 2H), 6.89 (d, *J* = 8.6 Hz, 2H), 6.29 (s, 1H), 4.68∼4.32 (m, 1H), 3.43 (s, 1H), 2.89~2.54 (m, 4H), 2.40 (s, 2H), 2.14 (s, 4H), 2.05∼1.83 (m, 3H), 1.82∼1.47 (m, 8H), 1.37∼1.23 (m, 4H).

### Example 3: Preparation of 1-(4-((1-cyclobutylpiperidin-4-yl)oxy)phenyl)-3-(2-morpholinoethyl)urea (BIOS-A-3)

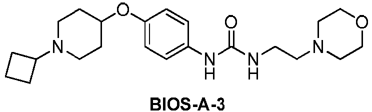

The synthesis route is as follows:

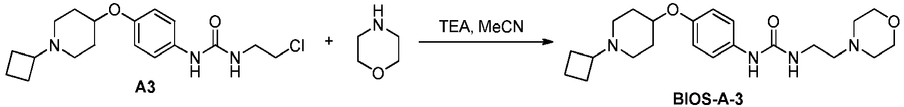

Intermediate A3 (100 mg, 0.284 mmol), morpholine (100 µL, 0.853 mmol), and triethylamine (197 µL) were dissolved in 4 mL acetonitrile and reacted in 80 °C oil bath for 2-3 hours. The reaction of the raw material was complete as detected by thin layer chromatography.

The reaction solution was concentrated to dryness and the crude product was purified on a preparative plate (petroleum ether: ethyl acetate: triethylamine = 1: 4: 1), affording 48 mg white solid (compound BIOS-A-3), with a yield of 41.99%.
MS:403.1[M+H]⁺_{∘}
¹H NMR (400 MHz, DMSO-*d*₆) δ = 8.43 (s, 1H), 7.25 (d, *J* = 9.0 Hz, 2H), 6.81 (d, *J* = 9.0 Hz, 2H), 5.99 (t, *J* = 5.4 Hz, 1H), 4.26∼4.16 (m, 1H), 3.59 (t, *J* = 4.6 Hz, 4H), 3.18 (q, *J* = 6.2 Hz, 2H), 2.74∼2.61 (m, 1H), 2.60∼2.52 (m, 2H), 2.37 (t, *J* = 5.6 Hz, 6H), 2.07∼1.83 (m, 6H), 1.83∼1.69 (m, 2H), 1.64∼1.50 (m, 4H).

### Example 4: Preparation of 1-(4-((1-cyclobutylpiperidin-4-yl)oxy)phenyl)-3-(2-(piperidin-1-yl)ethyl) urea (BIOS-A-4)

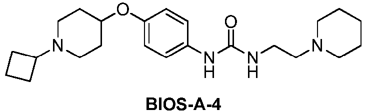

The synthesis route is as follows:

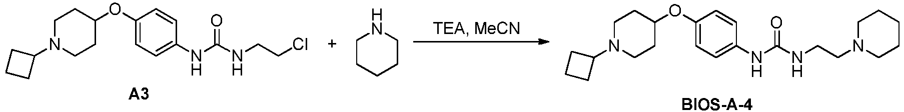

Referred to the synthesis route of compound BIOS-A-3 in Example 3, in this example, piperidine was used instead of morpholine from Example 3, and the remaining preparation steps were the same as in Example 3. 76 mg white solid (compound BIOS-A-4) was obtained with a yield of 66.81%.
MS:401.2[M+H]⁺_{∘}
¹H NMR (400 MHz, DMSO-*d*₆) δ 8.57 (s, 1H), 7.26 (d, *J* = 8.8 Hz, 2H), 6.81 (d, *J* = 8.8 Hz, 2H), 6.13 (s, 1H), 4.28∼4.16 (m, 1H), 3.23∼3.13 (m, 2H), 2.82∼2.69 (m, 1H), 2.60 (d, *J* = 5.4 Hz, 2H), 2.47∼2.36 (m, 6H), 2.09∼2.06 (m, 2H), 2.02∼1.85 (m, 4H), 1.85∼1.73 (m, 2H), 1.65∼1.50 (m, 8H), 1.45∼1.34 (m, 2H).

### Example 5: Preparation of 1-(4-((1-cyclobutylpiperidin-4-yl)oxy)phenyl)-3-(2-(pyrrolidin-1-yl)ethyl)urea (BIOS-A-5)

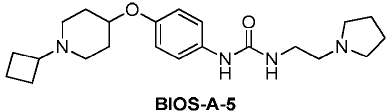

The synthesis route is as follows:

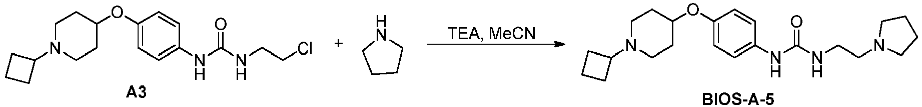

Referred to the synthesis route of compound BIOS-A-3 in Example 3, in this example, pyrrolidine was used instead of morpholine from Example 3, and the remaining preparation steps were the same as in Example 3. 31 mg white solid (compound BIOS-A-5) was obtained with a yield of 29.15%.
MS:401.2[M+H]⁺_{∘}
¹H NMR (400 MHz, DMSO-*d*₆) δ 8.42 (s, 1H), 7.25 (d, *J* = 8.8 Hz, 2H), 6.80 (d, *J* = 8.8 Hz, 2H), 6.00 (t, *J* = 5.4 Hz, 1H), 4.26∼4.14 (m, 1H), 3.21∼3.14 (m, 2H), 2.72∼2.62 (m, 1H), 2.61∼2.52 (m, 2H), 2.50∼2.37 (m, 8H), 2.05∼1.91 (m, 4H), 1.79∼1.48 (m, 10H).

### Example 6: Preparation of (R)-1-(4-((1-cyclobutylpiperidin-4-yl)oxy)phenyl)-3-(2-(2-methylpyrrolid in-1-yl)ethyl)urea (BIOS-A-6)

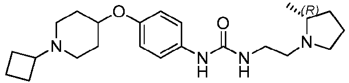

### BIOS-A-6

The synthesis route is as follows:

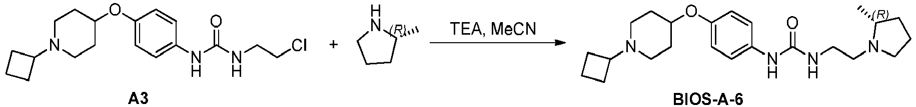

Referred to the synthesis route of compound BIOS-A-3 in Example 3, in this example, (*R*)-2-methylpyrrolidine was used instead of morpholine from Example 3, and the remaining preparation steps were the same as in Example 3. 74 mg yellow solid (compound BIOS-A-6) was obtained with a yield of 65.05%.
MS:387.1[M+H]⁺_{∘}
¹H NMR (400 MHz, DMSO-*d*₆) δ 8.50 (s, 1H), 7.26 (d, *J* = 8.6 Hz, 2H), 6.81 (d, *J* = 8.6 Hz, 2H), 6.05 (s, 1H), 4.23 (s, 1H), 2.89 (d, *J* = 9.3 Hz, 2H), 2.76 (t, *J* = 7.8 Hz, 1H), 2.61 (s, 2H), 2.45 (s, 2H), 2.21 (d, *J* = 9.0 Hz, 2H), 2.14∼2.04 (m, 2H), 2.02∼1.86 (m, 6H), 1.85∼1.50 (m, 6H), 1.40∼1.30 (m, 1H), 1.16∼1.03 (m, 5H).

### Example 7: Preparation of 1-(4-((1-cyclobutylpiperidin-4-yl)oxy)phenyl)-3-(2-(4,4-difluoropiperidin -1-yl)ethyl)urea (BIOS-A-7)

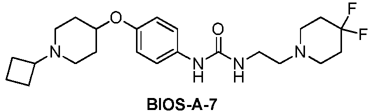

The synthesis route is as follows:

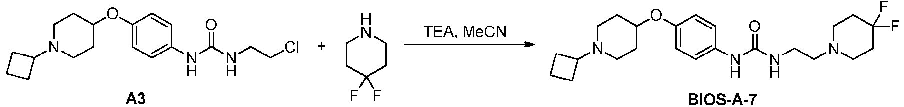

Referred to the synthesis route of compound BIOS-A-3 in Example 3, in this example, 4,4-difluoropiperidine was used instead of morpholine from Example 3, and the remaining preparation steps were the same as in Example 3. 100 mg pale white solid (compound BIOS-A-7) was obtained with a yield of 80.66%.
MS:437.2[M+H]⁺_{∘}
¹H NMR (400 MHz, DMSO-*d*₆) δ 8.52 (s, 1H), 7.28 (d, *J* = 9.0 Hz, 2H), 6.85 (d, *J* = 8.6 Hz, 2H), 6.07 (s, 1H), 4.37 (s, 1H), 3.19 (q, *J* = 6.2 Hz, 2H), 3.10∼3.00 (m, 1H), 2.85 (s, 2H), 2.60∼2.50 (m, 7H), 2.48∼2.42 (m, 2H), 2.20∼1.87 (m, 10H), 1.74∼1.55 (m, 3H).

### Example 8: Preparation of 1-(4-((1-cyclobutylpiperidin-4-yl)oxy)phenyl)-3-(2-thiomorpholinoethyl)urea (BIOS-A-8)

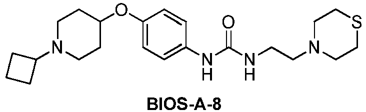

The synthesis route is as follows:

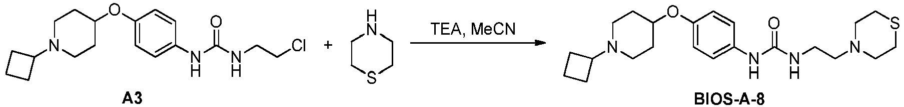

Referred to the synthesis route of compound BIOS-A-3 in Example 3, in this example, thiomorpholine was used instead of morpholine from Example 3, and the remaining preparation steps were the same as in Example 3. 73 mg white solid (compound BIOS-A-8) was obtained with a yield of 61.41%.
MS:419.2[M+H]⁺_{∘}
¹H NMR (400 MHz, DMSO-*d*₆) δ 8.39 (s, 1H), 7.25 (d, *J* = 9.0 Hz, 2H), 6.81 (d, *J* = 9.0 Hz, 2H), 5.93 (t, *J* = 5.4 Hz, 1H), 4.27∼4.16 (m, 1H), 3.16 (q, *J* = 6.1 Hz, 2H), 2.79∼2.54 (m, 11H), 2.40 (t, *J* = 6.4 Hz, 2H), 2.14∼1.84 (m, 6H), 1.84∼1.71 (m, 2H), 1.68∼1.49 (m, 4H).

### Example 9: Preparation of 1-(4-((1-cyclobutylpiperidin-4-yl)oxy)phenyl)-3-(2-(4-hydroxypiperidin-1 -yl)ethyl)urea (BIOS-A-9)

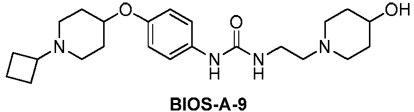

The synthesis route is as follows:

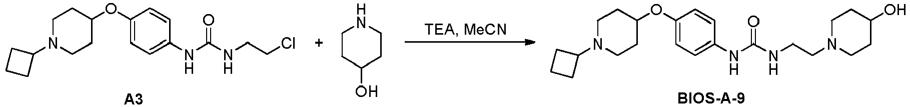

Referred to the synthesis route of compound BIOS-A-3 in Example 3, in this example, 4-hydroxypiperidine was used instead of morpholine from Example 3, and the remaining preparation steps were the same as in Example 3. 107 mg white solid (compound BIOS-A-9) was obtained with a yield of 90.45%.
MS:417.2[M+H]⁺_{∘}
¹H NMR (400 MHz, DMSO-*d*₆) δ 8.47 (s, 1H), 7.26 (d, *J* = 9.0 Hz, 2H), 6.82 (d, *J* = 9.0 Hz, 2H), 5.98 (s, 1H), 4.60 (s, 1H), 4.24 (s, 1H), 3.47 (s, 1H), 3.18 (d, *J* = 5.9 Hz, 2H), 2.82∼2.42 (m, 5H), 2.40 (s, 2H), 2.24∼1.68 (m, 12H), 1.67∼1.35 (m, 6H).

### Example 10: Preparation of 1-(4-((1-cyclobutylpiperidin-4-yl)oxy)phenyl)-3-(2-(4-methylpiperazin-1 -yl)ethyl)urea (BIOS-A-10)

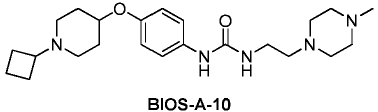

The synthesis route is as follows:

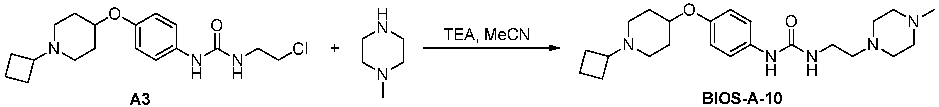

Referred to the synthesis route of compound BIOS-A-3 in Example 3, in this example, 4-methylpiperazine was used instead of morpholine from Example 3, and the remaining preparation steps were the same as in Example 3. 103 mg white solid (compound BIOS-A-10) was obtained with a yield of 87.27%.
MS:416.2[M+H]⁺_{∘}
¹H NMR (400 MHz, DMSO-*d*₆) δ 8.46 (s, 1H), 7.25 (d, *J* = 9.0 Hz, 2H), 6.81 (d, *J* = 9.0 Hz, 2H), 5.95 (t, *J* = 5.4 Hz, 1H), 4.26∼4.16 (m, 1H), 2.98∼2.90 (m, 2H), 2.77∼2.65 (m, 1H), 2.58 (t, *J* = 5.9 Hz, 2H), 2.40∼2.25 (m, 8H), 2.19 (s, 2H), 2.16 (s, 3H), 2.08∼1.84 (m, 6H), 1.84∼1.71 (m, 2H), 1.67∼1.48 (m, 4H).

### Example 11: Preparation of 1-(4-((1-cyclobutylpiperidin-4-yl)oxy)phenyl)-3-(2-(4-isopropylpiperazi n-1-yl)ethyl)urea (BIOS-A-11)

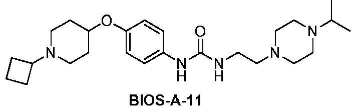

The synthesis route is as follows:

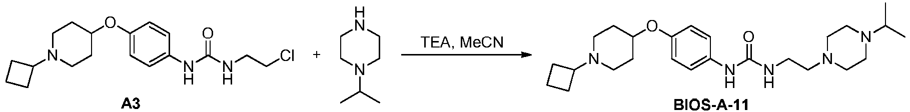

Referred to the synthesis route of compound BIOS-A-3 in Example 3, in this example, 4-isopropylpiperazine was used instead of morpholine from Example 3, and the remaining preparation steps were the same as in Example 3. 114 mg pale yellow solid (compound BIOS-A-11) was obtained with a yield of 90.48%.
MS: 444.3[M+H]⁺_{∘}
¹H NMR (400 MHz, DMSO-*d*₆) δ 8.44 (s, 1H), 7.25 (d, *J* = 9.0 Hz, 2H), 6.81 (d, *J* = 9.0 Hz, 2H), 5.94 (t, *J* = 5.5 Hz, 1H), 4.23 (s, 1H), 3.17 (q, *J* = 6.0 Hz, 2H), 2.84~2.54 (m, 5H), 2.48∼2.27 (m, 7H), 2.17∼1.69 (m, 9H), 1.67∼1.49 (m, 4H), 1.06∼0.88 (m, 7H).

### Example 12: Preparation of 1-(4-((1-cyclobutylpiperidin-4-yl)oxy)phenyl)-3-(2-(3-hydroxypiperidin-1-yl)ethyl)urea (BIOS-A-12)

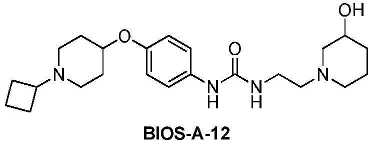

The synthesis route is as follows:

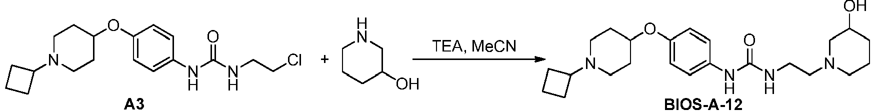

Referred to the synthesis route of compound BIOS-A-3 in Example 3, in this example, 3-hydroxypiperidine was used instead of morpholine from Example 3, and the remaining preparation steps were the same as in Example 3. 92 mg white solid (compound BIOS-A-12) was obtained with a yield of 77.77%.
MS:417.2[M+H]⁺_{∘}
¹H NMR (600 MHz, DMSO-*d*₆) δ 8.40 (s, 1H), 7.24 (d, *J* = 9.0 Hz, 2H), 6.80 (d, *J* = 9.0 Hz, 2H), 5.92 (t, *J* = 5.4 Hz, 1H), 4.58 (d, *J* = 50 Hz, 1H), 4.24∼4.16 (m, 1H), 3.51∼3.44 (m, 1H), 3.15 (q, *J* = 6.1 Hz, 2H), 2.82 (d, *J* = 10.8 Hz, 1H), 2.73∼2.62 (m, 2H), 2.57 (s, 2H), 2.41∼2.30 (m, 2H), 2.07∼1.92 (m, 4H), 1.91∼1.82 (m, 3H), 1.81∼1.71 (m, 4H), 1.65∼1.50 (m, 5H), 1.46∼1.36 (m, 1H), 1.13∼1.03 (m, 1H).

### Example 13: Preparation of 1-(4-((1-cyclobutylpiperidin-4-yl)oxy)phenyl)-3-(2-(N-methylhomopiper azin-1-yl)ethyl)urea (BIOS-A-13)

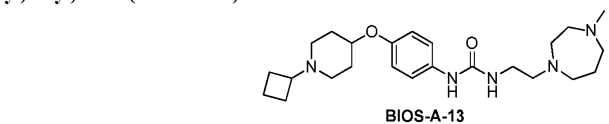

The synthesis route is as follows:

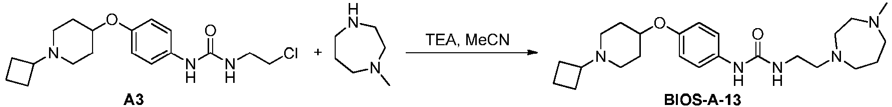

Referred to the synthesis route of compound BIOS-A-3 in Example 3, in this example, N-methylhomopiperazine was used instead of morpholine from Example 3, and the remaining preparation steps were the same as in Example 3. 84 mg white solid (compound BIOS-A-13) was obtained with a yield of 68.85%.
MS: 430.2[M+H]⁺_{∘}
¹H NMR (400 MHz, DMSO-*d*₆) δ 8.49 (s, 1H), 7.25 (d, *J* = 9.0 Hz, 2H), 6.81 (d, *J* = 9.0 Hz, 2H), 5.99 (s, 1H), 4.26∼4.15 (m, 1H), 3.17∼3.03 (m, 5H), 2.78∼2.63 (m, 10H), 2.35 (s, 3H), 2.31 (s, 2H), 2.09∼1.70 (m, 12H).

### Example 14: Preparation of 1-(4-((1-cyclobutylpiperidin-4-yl)oxy)phenyl)-3-(2-(piperazin-1-yl)ethyl)urea (BIOS-A-14)

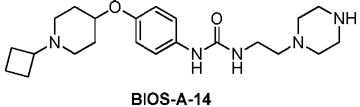

The synthesis route is as follows:

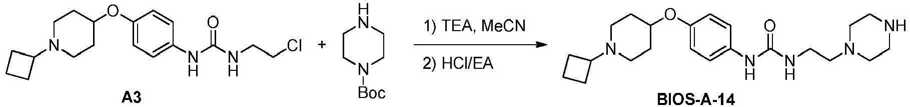

Intermediate A3 (100 mg, 0.284 mmol), 1-Boc-piperazine (158 mg, 0.853 mmol), and 197 µL triethylamine were dissolved in 4 mL acetonitrile and reacted in 80 °C oil bath for 2-3 hours. The reaction of the raw material was complete as detected by thin layer chromatography.

The reaction solution was concentrated to dryness and the crude product was purified on a preparative plate (petroleum ether: ethyl acetate: triethylamine = 1: 4: 1), affording 96 mg white solid as the intermediate. After reacting in 3 ml HCl/EA with the intermediate for 2 hours, 91mg white solid (hydrochloride of compound BIOS-A-14) was obtained, with a two-step yield of 62.71%
MS: 402.2[M+H]⁺_{∘}
¹H NMR (400 MHz, DMSO-*d*₆) δ 9.87 (s, 1H), 8.99 (d, *J* = 10.2 Hz, 1H), 7.32 (d, *J* = 9.0 Hz, 2H), 6.90 (d, *J* = 9.0 Hz, 2H), 6.68 (s, 1H), 3.63∼3.55 (m, 2H), 3.54∼3.45 (m, 4H), 3.24 (t, *J* = 6.2 Hz, 2H), 3.19∼3.13 (m, 8H), 2.96~2.76 (m, 2H), 2.41 (t, *J* = 10.2 Hz, 2H), 2.16∼2.12 (m, 4H), 2.04∼1.85 (m, 2H), 1.80∼1.61 (m, 2H).

### Example 15: Preparation of 1-(2-(4-cyclobutylpiperazin-1-yl)ethyl)-3-(4-((1-cyclobutylpiperidin-4-yl) oxy)phenyl)urea (BIOS-A-15)

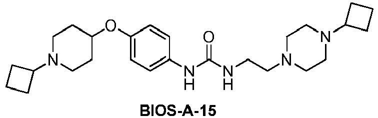

The synthesis route is as follows:

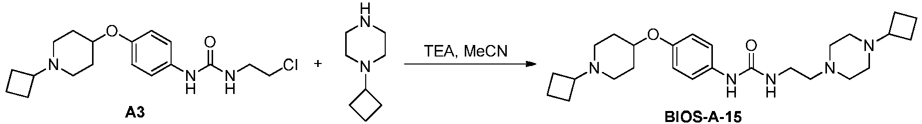

Referred to the synthesis route of compound BIOS-A-3 in Example 3, in this example, 1-cyclobutylpiperazine was used instead of morpholine from Example 3, and the remaining preparation steps were the same as in Example 3. 84 mg white solid (compound BIOS-A-15) was obtained with a yield of 64.91%.
MS: 456.3[M+H]⁺_{∘}
¹H NMR (400 MHz, DMSO-*d*₆) δ 8.45 (s, 1H), 7.26 (d, *J* = 8.6 Hz, 2H), 6.83 (d, *J* = 8.6 Hz, 2H), 5.95 (s, 1H), 4.27 (s, 1H), 3.18 (d, *J* = 6.1 Hz, 2H), 2.08~2.99(m, 1H), 2.86~2.58(m, 4H), 2.42∼2.36 (m, 9H), 2.09∼1.73 (m, 12H), 1.66∼1.62 (m, 6H).

### Example 16: Preparation of 1-(4-((1-cyclobutylpiperidin-4-yl)oxy)phenyl)-3-isopropylurea (BIOS-A-16)

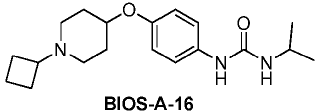

The synthesis route is as follows:

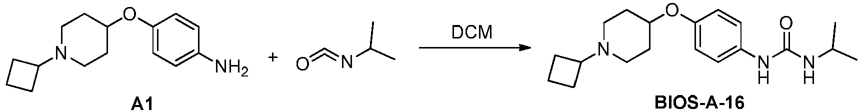

Referred to the synthesis route of compound BIOS-A-1 in Example 1, in this example, isopropyl isocyanate was used instead of cyclopentyl isocyanate from Example 1, and the remaining preparation steps were the same as in Example 1. 84 mg white solid (compound BIOS-A-16) was obtained with a yield of 62.42%.
MS: 456.3[M+H]⁺_{∘}
¹H NMR (400 MHz, DMSO-*d*₆) δ 8.61 (s, 1H), 7.29 (d, *J =* 8.6 Hz, 2H), 6.89 (d, *J =* 8.6 Hz, 2H), 6.29 (s, 1H), 4.68∼4.32 (m, 1H), 4.14∼4.09 (m, 1H), 3.43 (s, 1H), 2.05∼1.83 (m, 4H), 1.82∼1.47 (m, 6H), 1.37∼1.23 (m, 4H), 1.15 (d, *J* = 5.5 Hz, 6H).

### Example 17: Preparation of 1-(4-((1-cyclobutylpiperidin-4-yl)oxy)phenyl)-3-(2-(dimethylamino)ethy l)urea (BIOS-A-17)

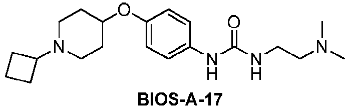

The synthesis route is as follows:

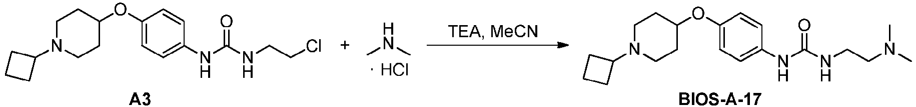

Referred to the synthesis route of compound BIOS-A-3 in Example 3, in this example, dimethylamine hydrochloride was used instead of morpholine from Example 3, and the remaining preparation steps were the same as in Example 3. 40 mg pale white solid (compound BIOS-A-17) was obtained with a yield of 39.07%.
MS: 437.2[M+H]⁺_{∘}
¹H NMR (400 MHz, DMSO-*d*₆) δ 8.52 (s, 1H), 7.28 (d, *J* = 9.0 Hz, 2H), 6.85 (d, *J* = 8.6 Hz, 2H), 6.07 (s, 1H), 4.37 (s, 1H), 3.10∼3.00 (m, 1H), 2.45 (t, *J* = 6.1 Hz, 2H), 2.24 (s, 6H), 2.20∼2.02 (m, 6H), 1.84∼1.55 (m, 10H).

### Example 18: Preparation of 1-(4-((1-cyclobutylpiperidin-4-yl)oxy)phenyl)-3-(2-(diethylamino)ethyl)urea (BIOS-A-18)

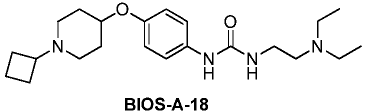

The synthesis route is as follows:

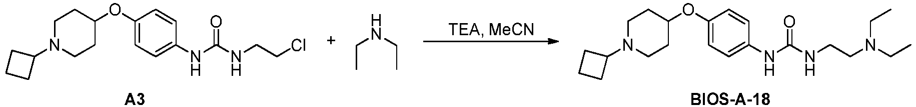

Referred to the synthesis route of compound BIOS-A-3 in Example 3, in this example, diethylamine was used instead of morpholine from Example 3, and the remaining preparation steps were the same as in Example 3. 67 mg white solid (compound BIOS-A-18) was obtained with a yield of 60.72%.
MS: 389.2[M+H]⁺_{∘}
¹H NMR (400 MHz, DMSO-*d*₆) δ 8.52 (s, 1H), 7.28 (d, *J* = 9.0 Hz, 2H), 6.85 (d, *J* = 8.6 Hz, 2H), 6.11 (s, 1H), 4.39 (s, 1H), 3.13∼3.02 (m, 1H), 2.70 (t, *J* = 5.8 Hz, 2H), 2.28∼2.21 (m, 4H), 2.20∼2.02 (m, 12H), 1.84∼1.55 (m, 10H).

### Example 19: Preparation of 1-(4-((1-cyclobutylpiperidin-4-yl)oxy)phenyl)-3-(2-(ethylamino)ethyl)urea (BIOS-A-19)

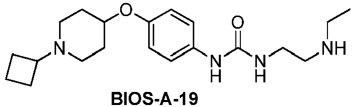

The synthesis route is as follows:

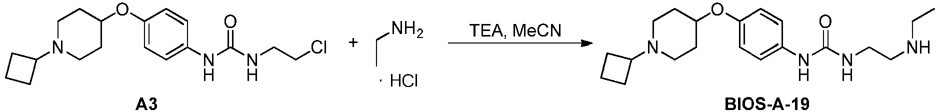

Referred to the synthesis route of compound BIOS-A-3 in Example 3, in this example, ethylamine hydrochloride was used instead of morpholine from Example 3, and the remaining preparation steps were the same as in Example 3. 76 mg white solid (compound BIOS-A-19) was obtained with a yield of 74.23%.
MS: 361.2[M+H]⁺_{∘}
¹H NMR (400 MHz, DMSO-*d*₆) δ 8.57 (s, 1H), 7.29 (d, *J* = 8.9 Hz, 2H), 6.88 (d, *J* = 8.9 Hz, 2H), 5.93 (t, *J* = 4.7 Hz, 1H), 4.37∼4.33 (m, 1H), 3.32 (q, *J* = 4.3 Hz, 2H), 2.89∼2.82 (m, 2H), 2.83∼2.66 (m, 6H), 1.98∼1.92 (m, 4H), 1.81∼1.74 (m, 2H), 1.77∼1.49 (m, 6H), 1.15 (t, *J* = 6.0 Hz, 3H).

### Example 20: Preparation of 1-(4-((1-cyclobutylpiperidin-4-yl)oxy)phenyl)-3-(2-morpholinoethyl)thiourea (BIOS-A-20)

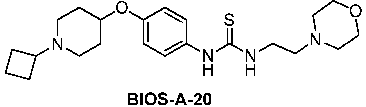

The synthesis route is as follows:

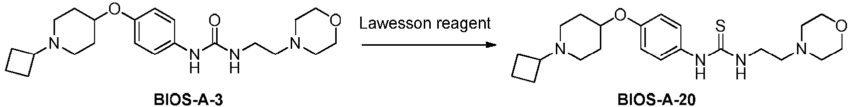

Compound BIOS-A-3 (100 mg, 0.248 mmol) and Lawesson Reagent (101 mg,0.248mmol) were dissolved in 4 mL anhydrous 1,4-dioxane and refluxed for 16 hours. The reaction of the raw material was complete as detected by thin layer chromatography.

The reaction solution was concentrated to dryness and purified on a preparative plate (petroleum ether: ethyl acetate: triethylamine = 5: 4: 1), affording 34 mg yellow solid (compound BIOS-A-20), with a yield of 32.75%.
MS: 419.1[M+H]⁺_{∘}
¹H NMR(400 MHz, DMSO-*d*₆) δ 9.18 (s, 1H), 7.25 (d, *J* = 9.0 Hz, 2H), 7.17 (t, *J* = 3.8 Hz, 1H), 6.81 (d, *J* = 9.0 Hz, 2H), 4.37∼4.32 (m, 1H), 3.69∼3.59 (m, 6H), 2.83∼2.77 (m, 1H), 2.77∼2.66 (m, 4H), 2.59 (t, *J* = 5.7 Hz, 2H), 2.52∼2.46 (m, 4H), 2.00∼1.90 (m, 4H), 1.81∼1.49 (m, 6H).

The synthesis route and preparation steps of compound BIOS-A-3 are described in Example 3 and will not be elaborated here.

### Example 21: Preparation of 1-(4-((1-cyclobutylpiperidin-4-yl)oxy)phenyl)-3-(2-(piperidin-1-yl)ethyl) thiourea (BIOS-A-21)

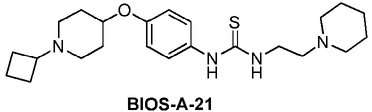

The synthesis route is as follows:

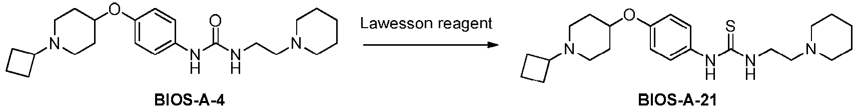

Referred to the synthesis route of compound BIOS-A-20 in Example 20, in this example, BIOS-A-4 was used instead of BIOS-A-3 from Example 20, and the remaining preparation steps were the same as in Example 20. 47 mg white solid (compound BIOS-A-21) was obtained with a yield of 45.49%.
MS: 417.1[M+H]⁺_{∘}
¹H NMR (400 MHz, DMSO-*d*₆) δ 8.59 (s, 1H), 7.27 (d, *J* = 8.9 Hz, 2H), 6.81 (d, *J* = 8.9 Hz, 2H), 6.13 (s, 1H), 4.28∼4.18 (m, 1H), 3.25∼3.15 (m, 2H), 2.82∼2.69 (m, 1H), 2.60 (d, *J* = 5.5 Hz, 2H), 2.47∼2.36 (m, 6H), 2.08 (t, *J* = 5.4Hz, 2H), 2.02∼1.85 (m, 4H), 1.85∼1.73 (m, 2H), 1.65∼1.50 (m, 8H), 1.45∼1.34 (m, 2H).

The synthesis route and preparation steps of compound BIOS-A-4 are described in Example 4 and will not be elaborated here.

### Example 22: Preparation of 1-(4-((1-cyclobutylpiperidin-4-yl)oxy)phenyl)-3-(2-(pyrrolidin-1-yl)ethy l)thiourea (BIOS-A-22)

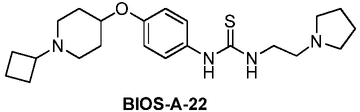

The synthesis route is as follows:

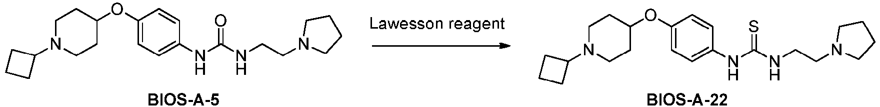

Referred to the synthesis route of compound BIOS-A-20 in Example 20, in this example, BIOS-A-5 was used instead of BIOS-A-3 from Example 20, and the remaining preparation steps were the same as in Example 20. 22 mg white solid (compound BIOS-A-22) was obtained with a yield of 22.03%.
MS: 403.1[M+H]⁺_{∘}
¹H NMR (400 MHz, DMSO-*d*₆) δ 8.47 (s, 1H), 7.26 (d, *J* = 8.8 Hz, 2H), 6.82 (d, *J* = 8.8 Hz, 2H), 6.01 (t, *J* = 5.4 Hz, 1H), 4.28∼4.16 (m, 1H), 3.21∼3.14 (m, 2H), 2.72∼2.62 (m, 1H), 2.61∼2.52 (m, 2H), 2.50∼2.37 (m, 8H), 2.05∼1.91 (m, 4H), 1.79∼1.48 (m, 10H).

The synthesis route and preparation steps of compound BIOS-A-5 are described in Example 5 and will not be elaborated here.

### Example 23: Preparation of (R)-1-(4-((1-cyclobutylpiperidin-4-yl)oxy)phenyl)-3-(2-(2-methylpyrroli din-1-yl)ethyl)thiourea (BIOS-A-23)

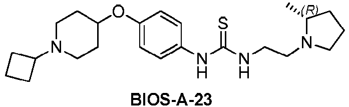

The synthesis route is as follows:

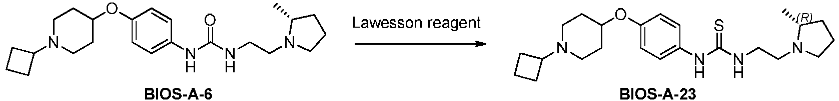

Referred to the synthesis route of compound BIOS-A-20 in Example 20, in this example, BIOS-A-6 was used instead of BIOS-A-3 from Example 20, and the remaining preparation steps were the same as in Example 20. 15 mg white solid (compound BIOS-A-23) was obtained with a yield of 14.52%.
MS: 417.1[M+H]⁺_{∘}
¹H NMR (400 MHz, DMSO-*d*₆) δ 8.58 (s, 1H), 8.20 (s, 2H), 7.29 (d, *J* = 8.6 Hz, 2H), 6.77 (d, *J* = 8.6 Hz, 2H), 6.03 (s, 1H), 4.21 (s, 1H), 2.82 (d, *J* = 9.3 Hz, 2H), 2.74 (t, *J* = 7.8 Hz, 1H), 2.61 (s, 2H), 2.45 (s, 2H), 2.21 (d, *J* = 9.0 Hz, 2H), 2.14∼2.04 (m, 2H), 2.02∼1.86 (m, 4H), 1.85∼1.50 (m, 6H), 1.40∼1.30 (m, 1H), 1.16∼1.03 (m, 5H).

The synthesis route and preparation steps of compound BIOS-A-6 are described in Example 6 and will not be elaborated here.

### Example 24: The antagonistic of compounds target to Histamine H3 receptor

The FLPPR assay (NIH Assay Guidance Manual: HTS Assay Validation-Section 4.3. Analysis (Potency), htpps://www.ncbi.nlm.nih.gov/books/NBK83783) was performed using SUVN-G3031 as the positive control, and 13 target compounds were tested for the antagonistic activity target to histamine H3 receptor. The results are shown in Table 1.

**Table 1. The antagonistic of compounds target to Histamine H3 receptor**

| **Compound ID** | **%Inh-AVERAGE @ 50 nM** | **%Inh-AVERAGE @ 5 nM** |
|---|---|---|
| Positive control SUVN-G3031 | 85.82 | 57.76 |
| BIOS-A-1 | 99.72 | 77.38 |
| BIOS-A-3 | 100.06 | 95.61 |
| BIOS-A-4 | 98.62 | 101.68 |
| BIOS-A-5 | 99.69 | 101.28 |
| BIOS-A-6 | 101.36 | 81.31 |
| BIOS-A-7 | 100.11 | 100.82 |
| BIOS-A-8 | 100.43 | 99.99 |
| BIOS-A-9 | 101.06 | 100.62 |
| BIOS-A-10 | 100.51 | 99.72 |
| BIOS-A-11 | 100.07 | 100.03 |
| BIOS-A-13 | 100.66 | 100.74 |
| BIOS-A-14 | 99.72 | 100.39 |
| BIOS-A-15 | 99.87 | 100.94 |

As shown in Table 1, some compounds show strong histamine H3 receptor antagonist activity, and most of them are more active than the positive control at 5 nM.

### Example 25:The hERG inhibition of compounds

In cardiomyocytes, inhibition of the delayed rectifier potassium current (IKr) channel protein encoded by the human Ether-a-go-go Related Gene (hERG) is the most importane mechanism by which drugs lead to prolongation of the QT interval. hERG is characterized by a specific molecular structure, which allows it to be inhibited by different structures, leading to severe arrhythmias. Statistically, 25-40% of lead compounds show varying degrees of hERG-related toxicity. Therefore, early evaluation of the effects of compounds on hERG is crucial in the drug development process.

The inhibition rate of compounds on hERG ion channels is examined by manual membrane clamp technique on HEK293 cell line which is stably expresses hERG ion channels. 100 nM of hERG inhibitor Cisapride is used as positive control.

The results are shown in Table 2. The inhibition rate of compounds, such as BIOS-A-13-BIOS-A-15, BIOS-A-3-BIOS-A-5, BIOS-A-10, BIOS-A-11 (10 µM), showed less than 5% on hERG channel, which indicated that the tested compounds basically perform have a good security..

**Table 2. The inhibition rate of compounds on hERG channel**

| Compound ID | Average inhibition rate (%) | |
|---|---|---|
| | 10 µM | 100 nM |
| Cisapride | ― | 86.6 |
| BIOS-A-3 | 1.56 | ― |
| BIOS-A-4 | 0.96 | ― |
| BIOS-A-5 | 2.64 | ― |
| BIOS-A-10 | -2.92 | ― |
| BIOS-A-11 | -1.22 | ― |
| BIOS-A-13 | -0.54 | ― |
| BIOS-A-14 | -0.62 | ― |
| BIOS-A-15 | -0.55 | ― |

| | | |
|---|---|---|
| Note:"-" refers not measured. | | |

### Example 26:The analgesic effects of compound BIOS-A-3

By exposing the sciatic nerve on male SD rats' (180-200 g) right hind leg, in the anterior segment where the sciatic nerve is about to bifurcate, sterile chromic gut thread (No. 4, 0.15 mm in diameter) was used to ligate 4 loops loosely, with each loop 1-2 mm apart, and the muscle and skin were sutured to establish the chronic constriction injury (CCI) model. In the sham-operated group, only the sciatic nerve was exposed without ligation, and the muscle and skin were sutured. The mechanical withdrawal threshold was detected by electronic analgesia meter (IITC-2391) on the 7^{th} day for 2 consecutive days.

The CCI model rats with stabilized mechanical withdrawal thresholds were randomly divided into three groups, named model group (model), pregabalin group (Pre, 30 mg/kg), BIOS-A-3 compound group (1 mg/kg), and were administered at 1 mL/100 g by gavage, and the same volume of saline was gavaged in Sham and model group. The basic value was determined before drug administration, and the mechanical withdrawal thresholds were detected at 0.5h, 1h, 2h, 4h and 6h after a single drug administration, and the p value was calculated by ANOVA with T-test. The results are shown in Figure 1.

The results showed that for the CCI neuropathic pain model in SD rats, the compound BIOS-A-3(1 mg/kg) significantly increased the mechanical withdrawal threshold of CCI model rats. The mechanical withdrawal thresholds at 0.5 h, 1 h and 6 h after administration were significantly different from that of the model group (P<0.05). The dose of 30 mg/kg of pregabalin only increased the mechanical withdrawal threshold of CCI rats at 0.5 h and 1 h after administration, which was significantly different from that of the model group, indicating that compound BIOS-A-3 had a longer acting time and was superior to pregabalin. Moreover, as time went on, the effect of compound BIOS-A-3 on the mechanical withdrawal threshold at a single time point was superior to that of pregabalin.

## Claims

1. A phenylurea derivative, which is a compound of general formula I or a pharmaceutically acceptable salt thereof: 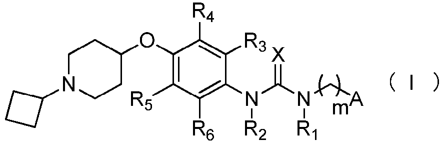 wherein:
X is selected from O or S;
m is selected from 0, 1, 2, 3, 4, 5 or 6;
A is selected from C₁₋₆ alkyl, -NR'R", substituted or unsubstituted 5-8 membered heterocyclyl, and substituted or unsubstituted 5-8 membered heteroaryl; wherein the heterocyclyl or heteroaryl has at least one substituent selected from one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₁ and R₂ are each independently selected from hydrogen, C₁₋₆ alkyl, -C(O)-alkyl or -S(O)₂-alkyl;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, R₅ and R₆ together with the carbon atoms on the benzene ring to which they are attached form a substituted or unsubstituted benzobicyclic structure, wherein the benzobicyclic structure can be but not limited to, a benzoheterocyclic ring;
the heterocyclyl, heteroaryl and benzoheterocyclic group contain at least one heteroatom, and the heteroatom is selected from N, O or S.

2. The phenylurea derivative according to claim 1, which is a compound of general formula II or a pharmaceutically acceptable salt thereof: 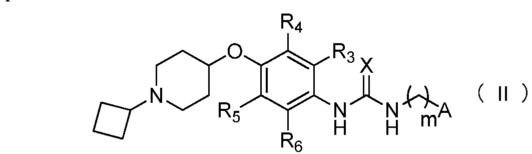 wherein:
X is selected from O or S;
m is selected from 0, 1, 2, 3, 4, 5 or 6;
A is selected from substituted or unsubstituted 5-8 membered heterocyclyl, and substituted or unsubstituted 5-8 membered heteroaryl; wherein the heterocyclyl or heteroaryl has at least one substituent selected from one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, R₅ and R₆ together with the carbon atoms on the benzene ring to which they are attached form a substituted or unsubstituted benzobicyclic structure, wherein the benzobicyclic structure can be but not limited to, a benzoheterocyclic ring;
the heterocyclyl, heteroaryl and benzoheterocyclic group contain at least one heteroatom, and the heteroatom is selected from N, O or S.

3. The phenylurea derivative according to claim 2, which is a compound of general formula II-1 or a pharmaceutically acceptable salt thereof: 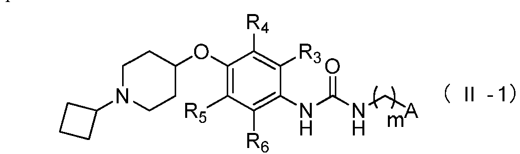 wherein:
m is selected from 0, 1, 2, 3, 4, 5 or 6;
A is selected from one of substituted or unsubstituted 6-membered heterocyclyl and substituted or unsubstituted 6-membered heteroaryl; wherein the heterocyclyl or heteroaryl has at least one substituent selected from one or more of C₁₋₆ alkyl, C₁₋₆ alkoxy, halogen, amino, hydroxyl, C₁₋₆ haloalkyl, C₁₋₆ alkylhydroxyl, C₁₋₆ alkylamino, C₁₋₆ haloalkoxy, C₁₋₆ alkoxyhydroxyl and C₁₋₆ alkoxy amino;
R₃, R₄, R₅, and R₆ are each independently selected from one or more of hydrogen, halogen, C₁₋₆ alkyl, amino, hydroxyl, C₁₋₆ haloalkyl, C₁₋₆ alkylhydroxyl and C₁₋₆ alkylamino;
the heterocyclyl and heteroaryl contain at least one heteroatom, and the heteroatom is selected from N, O or S.

4. The phenylurea derivative according to claim 1, which is a c compound of general formula III or a pharmaceutically acceptable salt thereof: 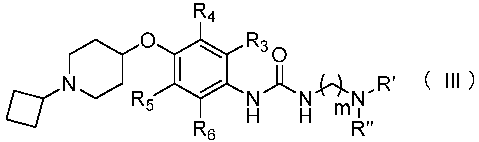 wherein:
m is selected from 0, 1, 2, 3, 4, 5 or 6;
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, R₅ and R₆ together with the carbon atoms on the benzene ring to which they are attached form a substituted or unsubstituted benzobicyclic structure, wherein the benzobicyclic structure can be but not limited to, a benzoheterocyclic ring;
the benzoheterocyclic group contains at least one heteroatom, and the heteroatom is selected from N, O or S.

5. The phenylurea derivative according to claim 1, which is a c compound of general formula IV or a pharmaceutically acceptable salt thereof: 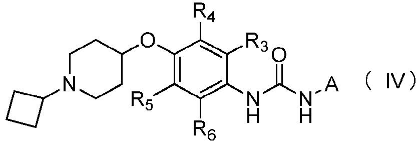 wherein:
A is selected from C₁₋₆ alkyl;
R₃, R₄, R₅, and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide;
or, R₃ and R₄ together with the carbon atoms on the benzene ring to which they are attached form a substituted or unsubstituted benzobicyclic structure, wherein the benzobicyclic structure can be but not limited to, a benzoheterocyclic ring;
or, R₅ and R₆ together with the carbon atoms on the benzene ring to which they are attached form a substituted or unsubstituted benzobicyclic structure, wherein the benzobicyclic structure can be but not limited to, a benzoheterocyclic ring;
the benzoheterocyclic group contains at least one heteroatom, and the heteroatom is selected from N, O or S.

6. A phenylurea derivative, which is a compound of the following structure, or an isomer thereof, or a solvate thereof, or a pharmaceutically acceptable salt thereof :
1-(4-((1-cyclobutylpiperidin-4-yl) oxy) phenyl)-3-cyclopentylurea;
1-(4-((1-cyclobutylpiperidin-4-yl)oxy)phenyl)-3-cyclohexylurea;
1-(4-((1-cyclobutylpiperidin-4-yl)oxy)phenyl)-3-(2-morpholinoethyl)urea;
1-(4-((1-cyclobutylpiperidin-4-yl)oxy)phenyl)-3-(2-(piperidin-1-yl)ethyl)urea;
1-(4-((1-cyclobutylpiperidin-4-yl)oxy)phenyl)-3-(2-(pyrrolidin-1-yl)ethyl)urea;
-1-(4-((1-cyclobutylpiperidin-4-yl)oxy)phenyl)-3-(2-(2-methylpyrrolidin-1-yl)ethyl)urea;
1-(4-((1-cyclobutylpiperidin-4-yl)oxy)phenyl)-3-(2-(4,4-difluoropiperidin-1-yl)ethyl)urea;
1-(4-((1-cyclobutylpiperidin-4-yl)oxy)phenyl)-3-(2-thiomorpholinoethyl)urea;
1-(4-((1-cyclobutylpiperidin-4-yl)oxy)phenyl)-3-(2-(4-hydroxypiperidin-1-yl)ethyl)urea;
1-(4-((1-cyclobutylpiperidin-4-yl)oxy)phenyl)-3-(2-(4-methylpiperazin-1-yl)ethyl)urea;
1-(4-((1-cyclobutylpiperidin-4-yl)oxy)phenyl)-3-(2-(4-isopropylpiperazin-1-yl)ethyl)urea;
1-(4-((1-cyclobutylpiperidin-4-yl)oxy)phenyl)-3-(2-(3-hydroxypiperidin-1-yl)ethyl)urea;
1-(4-((1-cyclobutylpiperidin-4-yl)oxy)phenyl)-3-(2-(N-Methylhomopiperazin-1-yl)ethyl)urea;
1-(4-((1-cyclobutylpiperidin-4-yl)oxy)phenyl)-3-(2-(piperazin-1-yl)ethyl)urea;
1-(2-(4-cyclobutylpiperazin-1-yl)ethyl)-3-(4-((1-cyclobutylpiperidin-4-yl)oxy)phenyl)urea;
1-(4-((1-cyclobutylpiperidin-4-yl)oxy)phenyl)-3-isopropylurea;
1-(4-((1-cyclobutylpiperidin-4-yl)oxy)phenyl)-3-(2-(dimethylamino)ethyl)urea;
1-(4-((1-cyclobutylpiperidin-4-yl)oxy)phenyl)-3-(2-(diethylamino)ethyl)urea;
1-(4-((1-cyclobutylpiperidin-4-yl)oxy)phenyl)-3-(2-(ethylamino)ethyl)urea;
1-(4-((1-cyclobutylpiperidin-4-yl)oxy)phenyl)-3-(2-morpholinoethyl)thiourea;
1-(4-((1-cyclobutylpiperidin-4-yl)oxy)phenyl)-3-(2-(piperidin-1-yl)ethyl)thiourea;
1 -(4-((1 -cyclobutylpiperidin-4-yl)oxy)phenyl)-3-(2-(pyrrolidin-1 -yl)ethyl)thiourea; or
1-(4-((1-cyclobutylpiperidin-4-yl)oxy)phenyl)-3-(2-(2-methylpyrrolidin-1-yl)ethyl)thiourea.

7. A phenylurea derivative, which is a compound of the following structure or a pharmaceutically acceptable salt thereof:
(*R*)-1-(4-((1-eyelobutylpiperidin-4-yl)oxy)phenyl)-3-(2-(2-methylpyrrolidin-1-yl)ethyl)urea; or
(*R*)-1-(4-((1-cyclobutylpiperidin-4-yl)oxy)phenyl)-3-(2-(2-methylpyrrolidin-1-yl)ethyl)thiourea.

8. A pharmaceutical composition, comprising at least one compound of any one of claims 1-7 or a pharmaceutically acceptable salt thereof, and at least one pharmaceutically acceptable carrier or excipient.

9. The compound of any one of claims 1-7 or the pharmaceutical composition of claim 8 for use in the manufacture of a medicament for preventing or treating a disease associated with histamine H3 receptor.

10. The compound or the pharmaceutical composition for use according to claim 9, wherein the medicament is used to prevent or treat cognitive impairment, dementia, attention deficit hyperactivity disorder, schizophrenia, epilepsy, sleep disorders, sleep apnea, obesity, eating disorders, pain or pruritus.

11. The compound or the pharmaceutical composition for use according to claim 9, wherein the medicament is used to prevent or treat neuropathic pain, which can be but not limited to, peripheral neuropathic pain or central neuropathic pain.
